# EUROPEAN PATENT APPLICATION

(11) **EP 0 722 725 A1**
(43) Date of publication of application: **24.07.1996**
(21) Application number: 95926507.5
(22) Date of filing: 27.07.1995
(51) Int. Cl.: A61K 31/215, A61K 31/38, A61K 31/557, A61K 31/695

(54) **VASCULAR SMOOTH MUSCLE CELL MIGRATION INHIBITOR**

(30) Priority: 09.08.1994 JP 19940187250
(71) Applicant: TEIJIN LIMITED, Osaka-shi Osaka 541 (JP)
(72) Inventor: MASEGI, Tsukio Tokyo Research Center, Hino-shi Tokyo 191 (JP); TOMIMORI, Koji Tokyo Research Center, Hino-shi Tokyo 191 (JP); WATANABE, Kunihito Tokyo Research Center, Hino-shi Tokyo 191 (JP)
(74) Representative: Votier, Sidney David
(86) International application number: PCT/JP1995/001492
(87) International publication number: WO 1996/004903

(57) **Abstract**

A vascular smooth muscle cell migration inhibitor and a preventive or remedy for post-PTCA restenosis, each containing an isocarbacyclin represented by general formula (1) and/or enantiomer thereof as the active ingredient, wherein W represents o-, m- or p-phenylene, C₃-C₇ cycloalkylene or thiophenediyl; R¹ represents hydrogen, methyl, ethyl or vinyl; R² represents optionally substituted linear or branched C₃₋C₉ alkyl, alkenyl or alkynyl, or C₃-C₇ cycloalkyl; R³⁰ represents hydrogen, C₁-C₁₀ alkyl, phenyl, naphthyl, benzyl or a mono-equivalent cation; Z¹⁰ and Z²⁰ represent each independently hydrogen tri(C₁-C₇ hydrocarbyl)silyl or a group that forms an acetal or ester linkage together with the oxygen atom to which it is bonded; n represents 0 or 1; and m represents an integer of 0 to 4.

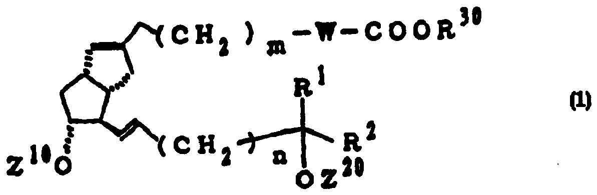

## Description

### Technical Field

This invention relates to a vascular smooth-muscle-cell migration inhibitor containing an isocarbacyclin which is useful as a drug inhibiting the hypertrophy and occlusion of a blood vessel as an active ingredient.

### Technical Background

Prostaglandins have various kinds of physiological activities such as strong platelet aggregation inhibition activity, vasodilation-based hypotensive activity, gastric acid secretion inhibiting activity, smooth muscle constricting activity, cell protecting activity and diuretic activity, and are useful compounds for the therapy or prevention of heart infarction, angina pectoris, arterial sclerosis, hypertension and duodenal ulcer and further for induction of delivery, abortion and the like.

Natural prostacyclin is a topical hormone produced mainly in the inner wall of the blood vessel of a living body and has strong physiological activities such as platelet aggregation inhibition activity and vasodilation activity Attempts have been made to provide this directly as a drug (P.J. Lewis, J. O. Grady, Clinical Pharmacology of Prostaglandin).

However, natural prostacyclin has an easily hydrolyzable enol-ether bond in its molecule so that easily deactivated under neutral or acidic conditions. Natural prostacyclin, therefor, can not be said to be a desirable compound as a drug due to its chemical instability. Under the circumstances, studies have been and are being diligently carried out for the synthesis of chemically stable synthetic prostacyclin derivatives having activities similar to those of natural prostacyclin (Synthesis, 1984, 449). Especially, 9(O)-methano-Δ^{6(9α)}-prostaglandin l₁s, as prostacyclins having sufficient chemical stability were synthesized by replacing the oxygen atom bound to 6 and 9 positions of natural prostacyclin by methylene group (-CH=). They have strong physiological activities comparable with natural prostacyclin such as platelet aggregation inhibition activity and vasodilation-based hypotensive activity, and are thought to be useful for drugs (JP-A-59-210044 and JP-A-61-197518).

On the other hand, percutaneous transluminal coronary angioplasty (later designated as "PTCA") brings low stress to a patient and exhibits excellent initial-treatment performance as a curing method for coronary artery diseases, and accordingly it has rapidly developed in recent years. It is said that its annual application exceeds 30,000 cases in Japan and reaches 300,000 cases in the United States. However, the largest disadvantage that 30-40% of the patients treated with PTCA are suffered from restenosis of coronary artery within several months after the operation remains unsolved. As for the mechanism of the development of post-PTCA restenosis, the explanation based on "vascular injury-to-response hypothesis" proposed by R. Ross is accepted in broad outline. The hypothesis is as follows. At first, the injury of vascular endothelial cells caused by some factors induces platelet adhesion and coagulation on the injured site, and this further stimulates the release of PDGF, cytokines and the like. By the released materials, the proliferation of vascular smooth muscle cell and the acceleration of the secretion of intercellular substances are secondarily caused, and finally arteriosclerosis is established. Therefor, trials for the prevention of post-PTCA restenosis are carried out by using an anti-platelet agent, an anticoagulant or the like. However, so far an agent having sufficient clinical effect is not reported. Further, although a compound capable of preventing the migration of vascular smooth muscle cell from intima to media, which is closely related to the mechanism of the development of restenosis, is strongly expected as an preventing agent for restenosis, a clinically effective agent having such activity is not reported until now.

### Disclosure of Invention

It is an object of the present invention to provide pharmaceutical agents for preventing post-PTCA restenosis.

It is another object of the present invention to provide effective pharmaceutical agents for preventing migration of vascular smooth muscle cells.

It is further another object of the present invention to provide agents containing an isocarbacyclin having a specific structure which shows the activity for inhibiting migration of vascular smooth muscle cells.

Other objects and advantages of the present invention will be apparent from the following description.

According to the present invention, the above objects and advantages of the present invention are achieved, first, by agents having the activity for inhibiting migration of vascular smooth muscle cells containing an isocarbacyclin expressed by the following formula (1), 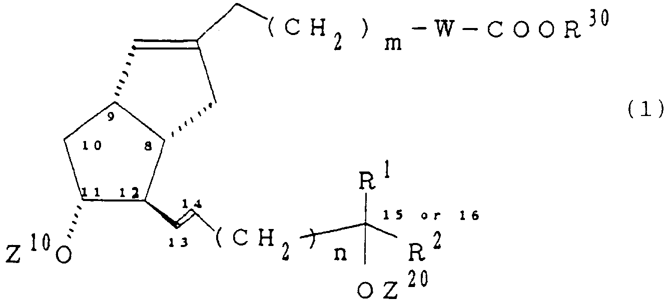 wherein W is an ortho, meta or para phenylene group, a C₃-C₇ cycloalkylene group or a thiophendiyl group; R¹ is a hydrogen atom, a methyl group, an ethyl group or a vinyl group; R² is a linear or branched C₃-C₉ alkyl group, alkenyl group or alkynyl group, or a C₃-C₇ cycloalkyl group each of which is optionally substituted; R³⁰is a hydrogen atom, a C₁-C₁₀ alkyl group, a phenyl group, a benzyl group, a naphthyl group or one equivalent of a cation; each of Z¹⁰ and Z²⁰ is independently a hydrogen atom, a tri(C₁-C₇ hydrocarbon)silyl group or a group which forms an acetal bond or an ester bond together with an oxygen atom to which it bonds; n is 0 or 1; and m is an integer of 0 to 4, and/or its enantiomer as an active ingredient.

### Preferred Embodiments of the Invention

lsocarbacyclins of the present invention are expressed by the above formula (1).

In the above formula (1), W is an ortho, meta or para phenylene group, a C₃-C₇ cycloalkylene or a thiophendiyl group. However, the ortho, meta or para phenylene group is especially preferable as W.

In the above formula (1), each of Z¹⁰ and Z²⁰ is independently a hydrogen atom, a tri(C₁-C₇ hydrocarbon)silyl group or a group which forms an acetal bond or an ester bond together with an oxygen atom to which it bonds.

Examples of the tri(C₁-C₇ hydrocarbon)silyl group as Z¹⁰ or Z²⁰ preferably include tri(C₁-C₄ alkyl)silyl groups such as trimethylsilyl, triethylsilyl triisopropylsilyl and t-butyldimethylsilyl groups; diphenyl(C₁-C₄ alkyl)silyl groups such as t-butyldiphenylsilyl group; di(C₁-C₄ alkyl)phenylsilyl groups such as a dimethylphenylsilyl group and a tribenzylsilyl group. Tri(C₁-C₄ alkyl)silyl and diphenyl(C₁-C₄ alkyl)silyl groups are preferred, and above all, a t-butyldimethylsilyl group is particularly preferred.

Examples of the group to form an acetal bond together with the oxygen atom to which it bonds include methoxymethyl, 1-ethoxyethyl, 2-methoxy-2-propyl, 2-ethoxy-2-propyl, (2-methoxyethoxy)methyl, benzyloxymethyl, 2-tetrahydro-pyranyl and 2-tetrahydrofuranyl groups. Among the above example, 2-tetrahydropyranyl, 2-tetrahydrofuranyl, 1-ethoxyethyl 2-ethoxy-2-propyl and (2-methoxyethoxy)-methyl groups are preferred, and above all, 2-tetrahydropyranyl group is particularly preferred.

Examples of the group to form an ester bond together with the oxygen atom to which it bonds include C₁-C₅ acyl groups such as formyl, acetyl, propionyl, butanoyl and pentanoyl groups, a benzoyl group and a toluoyl group. Acetyl and benzoyl groups are particularly preferred. Above all, a pair of hydrogen atoms are preferred when they take Z¹⁰ and Z²⁰ at the same time respectively.

In the above formula (1), R¹ is a hydrogen atom, a methyl group or a vinyl group. When n is 0, a hydrogen atom is preferred, and when n is 1, a methyl group is preferred.

In the above formula (1), R² is a linear or branched C₃-C₉ alkyl, alkenyl or alkynyl group or a C₃-C₇ cycloalkyl group each of which is optionally substituted.

Examples of the linear or branched not-substituted C₃-C₉ alkyl group as R² include n-propyl, iso-propyl, n-butyl, sec-butyl, tert-butyl, n-pentyl, n-hexyl, n-heptyl, n-octyl, n-decyl, 1-methylpentyl, 1-methylhexyl, 1,1-dimethylpentyl, 2-methylpentyl, 2-methylhexyl, 5-methylhexyl and 2,5-dimethylhexyl groups. Above all, n-butyl, n-pentyl, n-hexyl, (R)-, (S)- or (RS)-1-methylpentyl, (R)-, (S)- or (RS)-2-methylhexyl groups and the like are preferred. Examples of the C₃-C₉ alkyl moiety of the substituted C₃-C₉ alkyl group preferably include the same groups as shown above, and an example of the substituting moiety preferably includes a C₃-C₆ cycloalkyl group.

Examples of the linear or branched C₃-C₉ alkenyl group as R² include 2-butenyl, 2-pentenyl, 3-pentenyl, 2-hexenyl, 4-hexenyl, 2-methyl-4-hexenyl and 6-methyl-5-heptenyl groups.

Examples of the linear or branched C₃-C₉ alkynyl group as R² include 2-butynyl, 2-pentynyl, 3-pentynyl, 2-hexynyl, 4-hexynyl, 2-octynyl, 1-methyl-3-pentynyl, 1-methyl-3-hexynyl and 2-methyl-4-hexynyl groups.

Examples of the C₃-C₇ cycloalkyl group as R² include cyclopropyl, cyclobutyl, cyclopentyl, cyclohexyl, cycloheptyl groups. Above all, cyclopentyl and cyclohexyl groups and the like are preferred.

R³⁰ is a hydrogen atom, a linear or branched C₁-C₁₀ alkyl group, a phenyl group, a benzyl group, a naphthyl group or one equivalent of a cation. Examples of the C₁-C₁₀ alkyl group include methyl, ethyl, n-propyl, iso-propyl, n-butyl, sec-butyl, tert-butyl, n-pentyl, n-hexyl, n-heptyl, n-octyl, n-nonyl and n-decyl groups. Above all, a C₁-C₆ alkyl is preferred and a C₁-C₄ is especially preferred. Examples of the one equivalent of a cation include alkali metal cations such as Na⁺ and K⁺, divalent or trivalent metal cations such as 1/2Ca²⁺, 1/2Mg²⁺ and 1/3Al³⁺ and cations such as an ammonium ion and a tetramethylammonium ion. A hydrogen atom and a methyl group are preferred as the R³⁰, and a methyl group is especially preferred.

In the isocarbacyclins of the above formula (1), the steric configurations at 8, 9, 11, 12 and 15 positions (n=0) are identical to those of natural prostacyclin. Here, the numbers are expressed after the numbering in the natural prostacy-clin. The isocarbacyclins having such configurations are especially useful isomers. However, the isocarbacyclins of the present invention include not only those having such configurations but also their enantiomers and all isomers in terms of the asymmetric carbons.

Preferable specific examples of isocarbacyclins of the present invention will be listed below, wherein derivatives in which Z¹⁰, Z²⁰ and R³⁰are hydrogen atoms at the same time will be presented as typical compounds.
(1) (1S,5S,6S,7R)-3-(o-carboxybenzyl)-6-[(1E,3S)-3-hydroxy-1-octenyl]-7-hydroxybicyclo[3.3.0].2.octene
(2) (1S,5S,6S,7R)-3-(m-carboxybenzyl)-6-[(1E,3S)-3-hydroxy-1-octenyl]-7-hydroxybicyclo[3.3.0]-2-octene
(3) (1 S,5S,6S,7R)-3-(p-carboxybenzyl)-6-[(1E,3S)-3-hydroxy-1-octenyl]-7-hydroxybicyclo[3.3.0]-2-octene
(4) (1S,5S,6S,7R)-3-(o-carboxybenzyl)-6-[(1E,3S)-3-hydroxy-4-methyl-1-octenyl]-7-hydroxybicyclo[3.3.0]-2-octene
(5) (1 S,5S,6S,7R)-3-(o-carboxybenzyl)-6-[(1 E,3S)-3-hydroxy-4,4-dimethyl-1-octenyl]-7-hydroxybicyclo[3.3.0]-2-octene
(6) (1S,5S,6S,7R)-3-(o-carboxybenzyl)-6-[(1E,3S)-3-hydroxy-5-methyl-1-octenyl]-7-hydroxybicyclo[3.3.0]-2-octene
(7) (1S,5S,6S,7R)-3-(o-carboxybenzyl)-6-[(1E,3S)-3-hydroxy-5-methyl-1-nonenyl]-7-hydroxybicyclo[3.3.0]-2-octe-ne
(8) (lS,5S,6S,7R)-3-(o-carboxybenzyl)-6-[(lE,3S)-3-hydroxy-3-cyclopentyl-l-propenyl]-7-hydroxybicyclo[3.3.0]-2-octene
(9) (1 S,5S,6S,7R)-3-(o-carboxybenzyl)-6-[(1 E,3S)-3-hydroxy-3-cyclohexyl]-7-hydroxybicyclo[3.3.0]-2-octene
(10) (1S,5S,6S,7R)-3-(o-carboxybenzyl)-6-[(1E,3S)-4-hydroxy-4-methyl-1-octenyl]-7-hydroxybicyclo[3.3.0]-2-octene
(11) (1S,5S,6S,7R)-3-(o-carboxybenzyl)-6-[(1E,4R)-4-hydroxy-4-methyl-1-octenyl]-7-hydroxybicyclo[3.3.0]-2-octene
(12) (1 S,5S,6S,7R)-3-(o-carboxybenzyl)-6-[(1 E)-4-hydroxy-4-vinyl-1-octenyl]-7-hydroxybicyclo[3.3.0]-2-octene
(13) to (21) 3-(m-carboxybenzyl) derivatives of each of the compounds (4) to (12)
(22) to (30) 3-(p-carboxybenzyl) derivatives of each of the compounds (4) to (12)
(31) (1S,5S,6S,7R)-3-[2-(o-carboxyphenyl)ethyl]-6-[(1E,3S)-3-hydroxy-1-octenyl]-7-hydroxybicyclo[3.3.0]-2-octene
(32) (1S,5S,6S,7R)-3-[2-(o-carboxyphenyl)ethyl]-6-[(1E,3S)-3-hydroxy-4-methyl-1-octenyl]-7-hydroxybicyclo[3.3.-0]-2-octene
(33) (1S,5S,6S,7R)-3-[2-(o-carboxyphenyl)ethyl]-6-[(1E,3S)-3-hydroxy-4,4-dimethyl-1-octenyl]-7-hydroxybicyclo[-3.3.0]-2-octene
(34) (1S,5S,6S,7R)-3-[2-(o-carboxyphenyl)ethyl]-6-[(1 E,3S)-3-hydroxy-5-methyl-1-octenyl]-7-hydroxybicyclo[3.3.-0]-2-octene
(35) (1S 5S,6S,7R)-3-[2-(o-carbonxyphenyl)ethyl]-6-[(1E,3S)-3-hydroxy-5-methyl-1-nonenyl]-7-hydroxybicyclo[3.-3.0]-2-octene
(36) (1S,5S,6S,7R)-3-[2-(o-carboxyphenyl)ethyl]-6-[(1E,3S)-3-hydroxy-3-cyclopentyl-1-propenyl]-7-hydroxybicyclo[3.3.0]-2-octene
(37) (1S,5S,6S,7R)-3-[2-(o-carboxyphenyl)ethyl]-6-[(1E,3S)-3-hydroxy-3-cyclohexyl]-7-hydroxybicyclo[3.3.0]-2-octene
(38) (1S,5S,6S,7R)-3-[2-(o-carboxyphenyl)ethyl]-6-[(1 E,4S)-4-hydroxy-4-methyl-1-octenyl]-7-hydroxybicyclo[3.3.-0]-2-octene
(39) (1S,5S,6S,7R)-3-[2-(o-carboxyphenyl)ethyl]-6-[(1E,4R)-4-hydroxy-4-methyl-1-octenyl]-7-hydroxybicyclo[3.3.0]-2-octene
(40) (1S,5S,6S,7R)-3-[2-(o-carboxyphenyl)ethyl]-6-[(1E)-4-hydroxy-4-vinyl-1-octenyl]-7-hydroxybicyclo[3.3.0]-2-0-ctene
(41) to (50) 3-[2-(m-carboxyphenyl)ethyl] derivatives of each of the compounds (31) to (40)
(51) to (60) 3-[2-(p-carboxyphenyl)ethyl] derivatives of each of the compounds (31) to (40)
(61) (1S,5S,6S,7R)-3-[3-(o-carboxyphenyl)propyl]-6-[(1E,3S)-3-hydroxy-1-octenyl]-7-hydroxybicyclo[3.3.0]-2-octene
(62) 3-[3-(m-carboxyphenyl)propyl derivative of the compound (61)
(63) 3-[3-(p-carboxyphenyl)propyl derivative of the compound (61)
(64) (1S,5S,6S,7R)-3-[3-(o-carboxyphenyl)propyl]-6-[(1E,3S)-3-hydroxy-4-methyl-1-octenyl]-7-hydroxybicyclo[3.3-.0]-2-octene
(65) (1S,5S,6S,7R)-3-[3-(o-carboxyphenyl)propyl]-6-[(1E,3S)-3-hydroxy-4,4dimethyl-1-octenyl]-7-hydroxybicyclo[3.3.0]-2-octene
(66) (1S,5S,6S,7R)-3-[3-(o-carboxyphenyl)propyl]-6-[(1E,3S)-3-hydroxy-5-methyl-1-octenyl]-7-hydroxybicyclo[3.-3.0]-2-octene
(67) (1 S,5S,6S,7R)-3-[3-(o-carboxyphenyl)propyl]-6-[(1E,3S)-3-hydroxy-5-methyl-1-nonenyl]-7- hydroxybicyclo[3.-3.0]-2-octene
(68) (1S,5S,6S,7R)-3-[3-(o-carboxyphenyl)propyl]-6-[(1E,3S)-3-hydroxy-3-cyclopentyl-1-propenyl]-7-hydroxybicyclo[3.3.0]-2-octene
(69) (1S,5S,6S,7R)-3-[3-(o-carboxyphenyl)propyl]-6-[(1E,3S)-3-hydroxy-3-cyclohexyl]-7-hydroxybicyclo[3.3.0]-2-octene
(70) (1S,5S,6S,7R)-3-[3-(o-carboxyphenyl)propyl]-6-[(1E,4S)-4-hydroxy-4-methyl-1-octenyl]-7-hydroxybicyclo[3.-3.0]-2-octene
(71) (1S,5S,6S,7R)-3-[3-(o-carboxyphenyl)propyl]-6-[(1E,4R)-4-hydroxy-4-methyl-1-octenyl]-7-hydroxybicyclo[3.-3.0]-2-octene
(72) (1S,5S,6S,7R)-3-[3-(o-carboxyphenyl)propyl]-6-[(1E)-4-hydroxy-4-vinyl-1-octenyl]-7-hydroxybicyclo[3.3.0]-2-octene
(73) to (81) 3-[3-(m-carboxyphenyl)propyl derivatives of each of the compounds (64) to (72)
(82) to (90) 3-[3-(p-carboxyphenyl)propyl derivatives of each of the compounds (64) to (72)
(91) (1S,5S,6S,7R)-3-[4-(o-carboxyphenyl)butyl]-6-[(1E,3S)-3-hydroxy-1-octenyl]-7-hydroxybicyclo[3.3.0]-2-octene
(92) (1S,5S,6S,7R)-3-[4-(o-carboxyphenyl)butyl]-6-[(1E,3S)-3-hydroxy-4-methyl-1-octenyl]-7-hydroxybicyclo[3.3.-0]-2-octene
(93) (1S,5S,6S,7R)-3-[4-(o-carboxyphenyl)butyl]-6-[(1E,3S)-3-hydroxy-4,4-dimethyl-1-octenyl]-7-hydroxybicyclo[-3.3.0]-2-octene
(94) (1S,5S,6S,7R)-3-[4-(o-carboxyphenyl)butyl]-6-[(1E,3S)-3-hydroxy-5-methyl-1-octenyl]-7-hydroxybicyclo[3.3.-0]-2-octene
(95) (1S,5S,6S,7R)-3-[4-(o-carboxyphenyl)butyl]-6-[(1E,3S)-3-hydroxy-5-methyl-1-nonenyl]-7-hydroxybicyclo[3.3.-0]-2-octene
(96) (1S,5S,6S,7R)-3-[4-(o-carboxyphenyl)butyl]-6-[(1E,3S)-3-hydroxy-3-cyclopentyl-1-propenyl]- 7-hydroxybicy-clo[3.3.0]-2-octene
(97) (1S,5S,6S,7R)-3-[4-(o-carboxyphenyl)butyl]-6-[(1E,3S)-3-hydroxy-3-cyclohexyl]-7-hydroxybicyclo[3.3.0]-2-octene
(98) (1S,5S,6S,7R)-3-[4-(o-carboxyphenyl)butyl]-6-[(1E,4S)-4-hydroxy-4-methyl-1-octenyl]-7-hydroxybicyclo[3.3.-0]-2-octene
(99) (1S,5S,6S,7R)-3-[4-(o-carboxyphenyl)butyl]-6-[(1E,4R)-4-hydroxy-4-methyl-1-octenyl]-7-hydroxybicyclo[3.3.-0]-2-octene
(100) (1S,5S,6S,7R)-3-[4-(o-carboxyphenyl)butyl]-6-[(1E)-4-hydroxy-4-vinyl-1-octenyl]-7-hydroxybicyclo[3.3.0]-2-octene
(101) to (110) 3-[4-(m-carboxyphenyl)butyl derivatives of each of the compounds (91) to (100)
(111) to (120) 3-[4-(p-carboxyphenyl)butyl derivatives of each of the compounds (91) to (100)
(121) (1S,5S,6S,7R)-3-[5-(o-carboxyphenyl)pentyl]-6-[(1E,3S)-3-hydroxy-1-octenyl]-7-hydroxybicyclo[3.3.0]-2-octene
(122) (1S,5S,6S,7R)-3-[5-(o-carboxyphenyl)pentyl]-6-[(1E,3S)-3-hydroxy-4-methyl-1-octenyl]-7-hydroxybicyclo[3.-3.0]-2-octene
(123) (1S,5S,65,7R)-3-[5-(o-carboxyphenyl)pentyl]-6-[(1E,35)-3-hydroxy-4,4-dimethyl-1-octenyl]-7-hydroxybicyc-lo[3.3.0]-2-octene
(124) (1S,5S,6S,7R)-3-[5-(o-carboxyphenyl)pentyl]-6-[(1E,3S)-3-hydroxy-5-methyl-1-octenyl]-7-hydroxybicyclo[3.-3.0]-2-octene
(125)(1S,5S,6S,7R)-3-[5-(o-carboxyphenyl)pentyl]-6-[(1E,3S)-3-hydroxy-5-methyl-1-nonenyl]-7-hydroxybicyclo[3.-3.0]-2-octene
(126) (1S,5S,6S,7R)-3-[5-(o-carboxyphenyl)pentyl]-6-[(1E,3S)-3-hydroxy-3-cyclopentyl-1-propenyl]-7-hydroxybicy-clo[3.3.0]-2-octene
(127) (1S,5S,6S,7R)-3-[5-(o-carboxyphenyl)pentyl]-6-[(1E,3S)-3-hydroxy-3-cyclohexyl]-7- hydroxybicydo[3.3.0].2. octene
(128) (1S,5S,6S,7R)-3-[5-(o-carboxyphenyl)pentyl]-6-[(1E,4S)-3-hydroxy-4-methyl-1-octenyl]-7-hydroxybicyclo[3.-3.0]-2-octene
(129) (1S,5S,6S,7R)-3-[5-(o-carboxyphenyl)pentyl]-6-[(1E,4R)-4-hydroxy-4-methyl-1-octenyl]-7-hydroxybicyclo[3.-3.0]-2-octene
(130) (1S,5S,6S,7R)-3-[5-(o-carboxyphenyl)pentyl]-6-[(1E)-4-hydroxy-4-vinyl-1-octenyl]-7-hydroxybicyclo[3.3.0]-2-octene
(131) to (140) 3-[5-(m-carboxyphenyl)pentyl derivatives of each of the compounds (121) to (130)
(141) to (150) 3-[5-(p-carboxyphenyl)pentyl derivatives of each of the compounds (121) to (130)

The isocarbacyclins of the present invention include the above compounds and the like, while they are not limited to these.

The isocarbacyclins of the present invention are produced in the following process, that is, isocarbacyclins of the above formula (1) are produced by reacting 2,6,7-trisubstituted-3-methylene-bicyclo[3.3.0] octanes of the flowing formula (2), 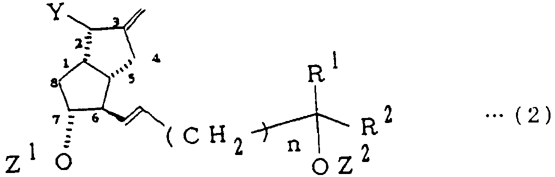 wherein Y is a group of (R⁴O)₂P(=B)A- or R⁵OC(=O)O- in which each of R⁴s is independently a C₁-C₆ hydrocarbon group, A and B are both oxygen atoms or one is an oxygen atom and the other is a sulfur atom, and R⁵ is a C₁-C₆ hydrocarbon group; R¹ is a hydrogen atom, a methyl group, an ethyl group or a vinyl group; R² is a linear or branched C₃-C₉ alkyl group, alkenyl group or alkynyl group, or a C₃-C₇ cycloalkyl group; each of Z¹ and Z² is independently a tri(C₁-C₇ hydrocarbon)silyl group or a group which forms an acetal bond or an ester bond together with an oxygen atom to which it bonds; and n is 0 or 1, with an organic zinc compound of the following formula (3),

X¹Zₙ-(CH₂)ₘ-W-COOR³ (3)

wherein R³ is a C₁-C₁₀ alkyl group, a phenyl group, a benzyl group or a naphthyl group; W is a phenylene group, a C₃-C₇ cycloalkylene group or a thiophendiyl group; X¹ is a halogen atom; and m is an integer of 0 to 4, in the presence of a cuprous salt of the following formula (4),

CuX² (4) (4)

wherein X² is a cyano group or a halogen atom, optionally subjecting the reaction product to a deprotection reaction and further optionally subjecting the reaction product to a salt-forming reaction.

The starting materials used in the process are 2,6,7-trisubstituted-3-methylenebicyclo[3.3.0]octane of the above formula (2) and an organic zinc compound of the above formula (3).

In the group (R⁴O)₂P(=B)A- out of the substituents represented by Y in the above formula (2) examples of the C₁-C₆ hydrocarbon group as R⁴ include methyl, ethyl, propyl and phenyl groups. Examples of the substituent Y preferably include diethoxyphosphoryloxy, dipropoxyphosphoryloxy diphenoxyphosphoryloxy dimethoxythiophosphoryloxy, diethoxythiophosphoryloxy, dimethoxyphosphorylthio and diethoxyphosphorylthio groups.

In the group R⁵OC(=O)O- out of the substituents represented by Y in the above formula (2) examples of the C₁-C₅ hydrocarbon group as R⁵ include methyl, ethyl, propyl, allyl, butyl, hexyl and phenyl groups. Examples of the substituent Y preferably include groups such as methoxycarbonyloxy group corresponding to the R⁵ groups described here.

Some of the 2,6,7-trisubstituted-3-methylenebicyclo[3.3.0]octanes of the above formula (2) used as starting materials of the process are known, and their production methods are described, for example, in the following Japanese Laid-open Patent Publications:
JP-A-62-61937
JP-A-62-258330
JP-A-63-303956
The compound expressed by the above formula (2) that are not described in these patents publications also can be produced in the similar method.

For the above purposes, the isocarbacyclins of the present invention can be applied by oral administration or by parenteral administration such as intrarectal, subcutaneous, intramuscular or intravenous administration, and above all, they are preferably applied by oral administration or intravenous administration.

For oral administration, it can be formulated into a solid preparation or a liquid preparation. Examples of the solid preparation include a tablet, a pill, powder or granules. In these solid preparations, an active ingredient is mixed with a pharmaceutically acceptable carrier such as sodium bicarbonate, calcium carbonate, potato starch, sucrose, mannitol or carboxymethylcellulose. Formulation is carried out by an ordinary process. However, an additive for formulation such as calcium stearate or magnesium stearate other than the above carrier is optionally incorporated to the solid preparation.

Enteric coating can be applied on the above solid preparation by spraying an organic solvent solution or an aqueous solution of an enteric substance such as cellulose acetate phthalate, hydroxypropylmethylcellulose phthalate, polyvinyl alcohol phthalate, styrene-maleic anhydride copolymer or methacrylic acid-methyl methacrylate copolymer to obtain an enteric preparation. A solid preparation such as powder or granules can be encapsulated with an enteric capsule.

Examples of the liquid preparation for oral administration include an emulsion, a solution, a suspension, a syrup and an elixir. These preparations can be incorporated with an ordinal, pharmaceutically acceptable carrier such as water or a liquid paraffin. An oily substrate such as coconut oil, fractionated coconut oil, soy bean oil or corn oil can be used as the carrier.

An ordinary additive such as an adjuvant, flavoring agent, stabilizer or antiseptic can optionally be used as the pharmaceutically acceptable carrier. Further, the liquid preparation can be administered also in a dosage form of a capsule made of an absorbable substance such as gelatin. Examples of the solid preparation for intrarectal administration include a suppository containing the active ingredient and prepared in an ordinary method.

A parenteral preparation is administered as a sterile aqueous or nonaqueous liquid, a suspension or an emulsion. The nonaqueous solution or suspension can be prepared using a pharmaceutically acceptable carrier such as propylene glycol, polyethylene glycol, a vegetable oil such as olive oil or soy bean oil, or an injectable organic ester such as ethyl oleate. These preparations also can be incorporated with an adjuvant such as an aseptic, humectant, emulsifier, dispersant or stabilizer. The solution, suspension and emulsion can be made aseptic by subjecting them to a treatment such as filtration by using a bacteria retaining filter, heating, treatment with a bactericidal agent or ultraviolet radiation. Further, a sterile solid preparation is prepared, and this can be administered after dissolving in a sterile water or a sterile injection medium just before use. Further, a homogeneous solution consisting of a vegetable oil such as soy bean oil, a phospholipid such as lecithin and an isocarbacyclin of the present invention is added with water, the obtained mixture is homogenized, for example, by a homogenizer such as a pressure injection homogenizer or an ultrasonic homogenizer to obtain a fat emulsion, and such an emulsion can be used as an injection.

Examples of the transcutaneous preparation include an ointment, a cream and the like, and these preparations can be produced by an ordinary process.

In the case where the drug of the present invention is used for inhibiting the hypertrophy of a blood vessel, dosage differs depending on the extent of the symptoms, age, sex and weight of a patient, administration rout and the like, but for a normal adult about 1 µg to 1 mg may be administered per day in terms of an isocarbacyclin component in it. The dosage may be administered once a day or divided into several times a day, for example, two to six times.

The characteristic features of the present invention described above are itemized as follows.
1. A pharmaceutical agent for preventing migration of vascular smooth cells containing an isocarbacyclin described by the following formula (1), 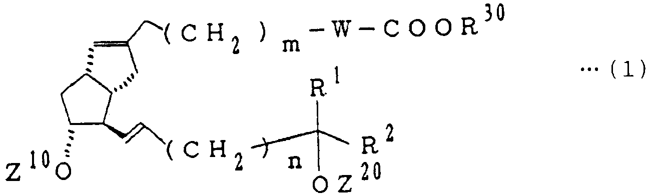 wherein W is an ortho, meta or para phenylene group, a C₃-C₇ cycloalkylene group or a thiophendiyl group; R¹ is a hydrogen atom, a methyl group, an ethyl group or a vinyl group; R² is a linear or branched C₃-C₉ alkyl group, alkenyl group or alkynyl group, or a C₃-C₇ cycloalkyl each of which is optionally substituted; R³⁰ is a hydrogen atom, a C₁-C₁₀ alkyl group, a phenyl group, a benzyl group, a naphthyl group or one equivalent of a cation; each of Z¹⁰ and Z²⁰ is independently a hydrogen atom, a tri(C₁-C₇ hydrocarbon)silyl group or a group which forms an acetal bond or an ester bond together with an oxygen atom to which it bonds; n is 0 or 1; and m is an integer of 0 to 4, and/or its enantiomer as an active ingredient.
2. A pharmaceutical agent for preventing migration of vascular smooth cells described in the above paragraph 1, wherein, in the above formula (1), W is an ortho, meta or para phenylene group, and Z¹⁰ and Z²⁰ are each a hydrogen atom.
3. A pharmaceutical agent for preventing migration of vascular smooth cells described in the above paragraph 1, wherein, in the above formula (1), R¹ is a hydrogen atom or a methyl group, R² is a linear or branched C₃-C₉ alkyl, or a C₃-C₉ alkyl group substituted with a C₂-C₆ cycloalkyl group, R³⁰is a hydrogen atom, a linear or branched C₁-C₁₀ alkyl group, or one equivalent of a cation, and m is 0 or 1.
4. A pharmaceutical agent for preventing migration of vascular smooth cells described in the above paragraph 2, wherein, in the above formula (1), R¹ is a hydrogen atom or a methyl group, R² is a linear or branched C₃-C₉ alkyl, or a C₃-C₉ alkyl group substituted with a C₃-C₆ cycloalkyl group, R³⁰ is a hydrogen atom or a linear or branched C₁-C₁₀ alkyl, or one equivalent of a cation, and m is 0 or 1.
5. A pharmaceutical agent for preventing migration of vascular smooth cells described in the above paragraph 1, wherein, in the above formula (1), n=0, and R¹ is a hydrogen atom.
6. A pharmaceutical agent for preventing migration of vascular smooth cells described in the above paragraph 2, wherein, in the above formula (1), n=0, and R¹ is a hydrogen atom.
7. A pharmaceutical agent for preventing migration of vascular smooth cells described in the above paragraph 3, wherein, in the above formula (1), n=0, and R¹ is a hydrogen atom.
8. A pharmaceutical agent for preventing migration of vascular smooth cells described in the above paragraph 4, wherein, in the above formula (1), n=0, and R¹ is a hydrogen atom.
9. A pharmaceutical agent for preventing migration of vascular smooth cells described in the above paragraph 1, wherein, in the above formula (1), n=1, and R¹ is a methyl group.
10. A pharmaceutical agent for preventing migration of vascular smooth cells described in the above paragraph 2, wherein, in the above formula (1), n=1, and R¹ is a methyl group.
11. A pharmaceutical agent for preventing migration of vascular smooth cells described in the above paragraph 3, wherein, in the above formula (1). n=1, and R¹ is a methyl group.
12. A pharmaceutical agent for preventing migration of vascular smooth cells described in the above paragraph 4, wherein, in the above formula (1), n=1, and R¹ is a methyl group.
13. A pharmaceutical agent for preventing migration of vascular smooth cells described in the above paragraph 1, wherein m=0 in the above formula (1).
14. A pharmaceutical agent for preventing migration of vascular smooth cells described in the above paragraph 2, wherein m=0 in the above formula (1).
15. A pharmaceutical agent for preventing migration of vascular smooth cells described in the above paragraph 3, wherein m=0 in the above formula (1).
16. A pharmaceutical agent for preventing migration of vascular smooth cells described in the above paragraph 4, wherein m=0 in the above formula (1).
17. A pharmaceutical agent for preventing migration of vascular smooth cells described in the above paragraph 5, wherein m=0 in the above formula (1).
18. A pharmaceutical agent for preventing migration of vascular smooth cells described in the above paragraph 6, wherein m=0 in the above formula (1).
19. A pharmaceutical agent for preventing migration of vascular smooth cells described in the above paragraph 7, wherein m=0 in the above formula (1).
20. A pharmaceutical agent for preventing migration of vascular smooth cells described in the above paragraph 8, wherein m=0 in the above formula (1).
21. A pharmaceutical agent for preventing migration of vascular smooth cells described in the above paragraph 9, wherein m=0 in the above formula (1).
22. A pharmaceutical agent for preventing migration of vascular smooth cells described in the above paragraph 10, wherein m=0 in the above formula (1).
23. A pharmaceutical agent for preventing migration of vascular smooth cells described in the above paragraph 11, wherein m=0 in the above formula (1).
24. A pharmaceutical agent for preventing migration of vascular smooth cells described in the above paragraph 12, wherein m=0 in the above formula (1).
25. A pharmaceutical agent for preventing migration of vascular smooth cells described in the above paragraph 1, wherein m=1 in the above formula (1).
26. A pharmaceutical agent for preventing migration of vascular smooth cells described in the above paragraph 2, wherein m=1 in the above formula (1).
27. A pharmaceutical agent for preventing migration of vascular smooth cells described in the above paragraph 3, wherein m=1 in the above formula (1).
28. A pharmaceutical agent for preventing migration of vascular smooth cells described in the above paragraph 4, wherein m=1 in the above formula (1).
29. A pharmaceutical agent for preventing migration of vascular smooth cells described in the above paragraph 5, wherein m=1 in the above formula (1).
30. A pharmaceutical agent for preventing migration of vascular smooth cells described in the above paragraph 6, wherein m=1 in the above formula (1).
31. A pharmaceutical agent for preventing migration of vascular smooth cells described in the above paragraph 7, wherein m=1 in the above formula (1).
32. A pharmaceutical agent for preventing migration of vascular smooth cells described in the above paragraph 8, wherein m=1 in the above formula (1).
33. A pharmaceutical agent for preventing migration of vascular smooth cells described in the above paragraph 9, wherein m=1 in the above formula (1).
34. A pharmaceutical agent for preventing migration of vascular smooth cells described in the above paragraph 10, wherein m=1 in the above formula (1).
35. A pharmaceutical agent for preventing migration of vascular smooth cells described in the above paragraph 11, wherein m=1 in the above formula (1).
36. A pharmaceutical agent for preventing migration of vascular smooth cells described in the .pa above paragraph 12, wherein m=1 in the above formula (1).
37. A pharmaceutical agent for preventing or curing post-PTCA restenosis containing an isocarbacyclin and/or its enantiomer described in the above formula (1) as an active ingredient.
38. A pharmaceutical agent for preventing or curing post-PTCA restenosis described in the above paragraph 37, wherein, in the above formula (1), W is an ortho, meta or para phenylene group, and Z¹⁰ and Z²⁰ are each a hydrogen atom.
39. A pharmaceutical agent for preventing or curing post-PTCA restenosis described in the above paragraph 37, wherein, in the above formula (1), R¹ is a hydrogen atom or a methyl group, R² is a linear or branched C₃-C₉ alkyl or a C₃-C₉ alkyl group substituted with a C₃-C₆ cycloalkyl group, R³⁰ is a hydrogen atom, a linear or branched C₁-C₁₀ alkyl, or one equivalent of a cation, and m is 0 or 1.
40. A pharmaceutical agent for preventing or curing post-PTCA restenosis described in the above paragraph 38, wherein, in the above formula (1), R¹ is a hydrogen atom or a methyl group, R² is a linear or branched C₃-C₉ alkyl or a C₃-C₉ alkyl group substituted with a C₃-C₆ cycloalkyl group, R³⁰ is a hydrogen atom, a linear or branched C₁-C₁₀ alkyl, or one equivalent of a cation, and m is 0 or 1.
41. A pharmaceutical agent for preventing or curing post-PTCA restenosis described in the above paragraph 37, wherein, in the above formula (1), n=0, and R¹ is a hydrogen atom.
42. A pharmaceutical agent for preventing or curing post-PTCA restenosis described in the above paragraph 38, wherein, in the above formula (1), n=0, and R¹ is a hydrogen atom.
43. A pharmaceutical agent for preventing or curing post-PTCA restenosis described in the above paragraph 39, wherein, in the above formula (1), n=0, and R¹ is a hydrogen atom.
44. A pharmaceutical agent for preventing or curing post-PTCA restenosis described in the above paragraph 40, wherein, in the above formula (1), n=0, and R¹ is a hydrogen atom.
45. A pharmaceutical agent for preventing or curing post-PTCA restenosis described in the above paragraph 37, wherein, in the above formula (1), n=1, and R¹ is a methyl group.
46. A pharmaceutical agent for preventing or curing post-PTCA restenosis described in the above paragraph 38, wherein, in the above formula (1), n=1, and R¹ is a methyl group.
47. A pharmaceutical agent for preventing or curing post-PTCA restenosis described in the above paragraph 39, wherein, in the above formula (1), n=1, and R¹ is a methyl group.
48. A pharmaceutical agent for preventing or curing post-PTCA restenosis described in the above paragraph 40, wherein, in the above formula (1), n=1, and R¹ is a methyl group.
49. A pharmaceutical agent for preventing or curing post-PTCA restenosis described in the above paragraph 37, wherein m=0 in the above formula (1).
50. A pharmaceutical agent for preventing or curing post-PTCA restenosis described in the above paragraph 38, wherein m=0 in the above formula (1).
51. A pharmaceutical agent for preventing or curing post-PTCA restenosis described in the above paragraph 39, wherein m=0 in the above formula (1).
52. A pharmaceutical agent for preventing or curing post-PTCA restenosis described in the above paragraph 40, wherein m=0 in the above formula (1).
53. A pharmaceutical agent for preventing or curing post-PTCA restenosis described in the above paragraph 41, wherein m=0 in the above formula (1).
54. A pharmaceutical agent for preventing or curing post-PTCA restenosis described in the above paragraph 42, wherein m=0 in the above formula (1).
55. A pharmaceutical agent for preventing or curing post-PTCA restenosis described in the above paragraph 43, wherein m=0 in the above formula (1).
56. A pharmaceutical agent for preventing or curing post-PTCA restenosis described in the above paragraph 44, wherein m=0 in the above formula (1).
57. A pharmaceutical agent for preventing or curing post-PTCA restenosis described in the above paragraph 45, wherein m=0 in the above formula (1).
58. A pharmaceutical agent for preventing or curing post-PTCA restenosis described in the above paragraph 46, wherein m=0 in the above formula (1).
59. A pharmaceutical agent for preventing or curing post-PTCA restenosis described in the above paragraph 47, wherein m=0 in the above formula (1).
60. A pharmaceutical agent for preventing or curing post-PTCA restenosis described in the above paragraph 48, wherein m=0 in the above formula (1).
61. A pharmaceutical agent for preventing or curing post-PTCA restenosis described in the above paragraph 37, wherein m=1 in the above formula (1).
62. A pharmaceutical agent for preventing or curing post-PTCA restenosis described in the above paragraph 38, wherein m=1 in the above formula (1).
63. A pharmaceutical agent for preventing or curing post-PTCA restenosis described in the above paragraph 39, wherein m=1 in the above formula (1).
64. A pharmaceutical agent for preventing or curing post-PTCA restenosis described in the above paragraph 40, wherein m=1 in the above formula (1).
65. A pharmaceutical agent for preventing or curing post-PTCA restenosis described in the above paragraph 41, wherein m=1 in the above formula (1).
66. A pharmaceutical agent for preventing or curing post-PTCA restenosis described in the above paragraph 42, wherein m=1 in the above formula (1).
67. A pharmaceutical agent for preventing or curing post-PTCA restenosis described in the above paragraph 43, wherein m=1 in the above formula (1).
68. A pharmaceutical agent for preventing or curing post-PTCA restenosis described in the above paragraph 44, wherein m=1 in the above formula (1).
69. A pharmaceutical agent for preventing or curing post-PTCA restenosis described in the above paragraph 45, wherein m=1 in the above formula (1).
70. A pharmaceutical agent for preventing or curing post-PTCA restenosis described in the above paragraph 46, wherein m=1 in the above formula (1).
71. A pharmaceutical agent for preventing or curing post-PTCA restenosis described in the above paragraph 47, wherein m=1 in the above formula (1).
72. A pharmaceutical agent for preventing or curing post-PTCA restenosis described in the above paragraph 48, wherein m=1 in the above formula (1).

### Examples

The present invention will be explained further in detail hereafter with Examples.
However, the invention is not limited to the examples. In the formulae in Examples, OZ¹ stands for a t-butyldimethylsilyloxy group, OZ² stands for a trimethylsilyloxy group, and OZ³ stands for a t-butyldiphenylsilyloxy group.

### Reference Example 1

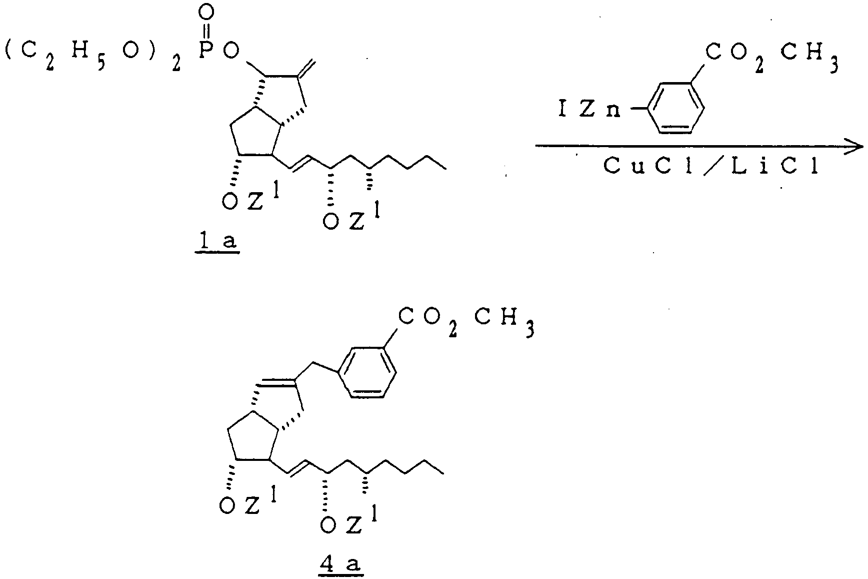

Zinc (0.850 g, 13 mmol) was placed in a 30-ml reactor, and after the reactor was flushed with argon gas, distilled tetrahydrofuran (2 ml) was added. Added thereto was 80 µl of 1,2-dibromoethane, the mixture was stirred under heating at 65°C for 1 minute, and further stirred at room temperature for 30 minutes. Then, 100 µl of trimethylchlorosilane was added, and the mixture was stirred at room temperature for 30 minutes. Thereafter, a solution of methyl-3-iodobenzoate (2.62 g, 10 mmol) in dry dimethylformamide (10 ml) was added, and the mixture was stirred at 40°C for 16 hours. A solution of cuprous chloride (0.990 g, 10 mmol) and lithium chloride (0.848 g, 20 mmol) in distilled tetrahydrofuran (10 ml) was prepared in a 50-ml reactor, and the above liquid mixture was added thereto at room temperature. The mixture was stirred at 30°C for 1 hour. Added to this liquid mixture was a solution of (1S,5R,6R,7R)-2-diethoxyphosphoryloxy-3-methylene-6-[(1E,3S,5S)-3-t-butyldimethylsilyloxy-5-methyl-1-noneyl]-7-t-butyldimethylsilyloxybicyclo[3.3.0]octane 1a (0.672 g, 1 mmol) in dry tetrahydrofuran (10 ml) at room temperature, and the mixture was stirred at 30°C for 2 hours. Added to the liquid mixture was 200 ml of a saturated ammonium chloride aqueous solution to terminate the reaction, and the reaction mixture was extracted with ethyl acetate (200 ml x 2 times). A separated organic layer was washed with an aqueous sodium chloride solution, dried over anhydrous magnesium sulfate, and concentrated to give a crude product (1.97 g). This product was subjected to silica gel chromatography (silica gel 40 g; elution liquid hexane:ethyl acetate = 20:1) to give an intended compound 4a (0.641 g, 0.98 mmol, yield 98%).

| | |
|---|---|
| ¹H-NMR (CDCl₃, ppm) δ : | 7.85(2H,m), 7.35(2H,m), 5.45(2H,m), 5.30(1 H,d,J=1.2Hz), 4.10(1 H,m), 3.90(3H, s), 3.70(1 H,m), 3.40(2H,bs), 3.00(1 H,m), 2.40-2.20(3H,m), 1.95-1.80(2H,m), 1.40-1.10(10H,m), 0.90-0.80(18H,m), 0.09(18H,s) |
| IR (liquid film) cm⁻¹: | 2955, 2920, 1725, 1607, 1590, 965, 735 |

### Reference Example 2

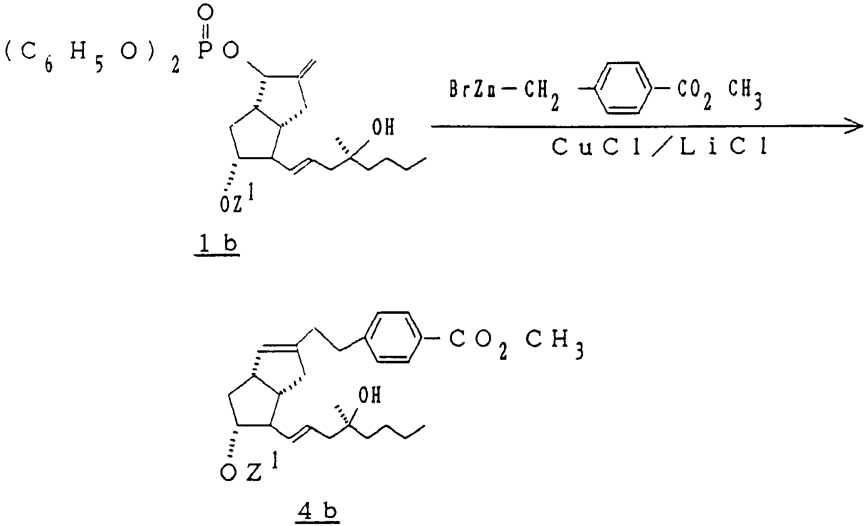

Zinc (0.850 g, 13 mmol) was placed in a 50-ml reactor, and after the reactor was flushed with argon gas, distilled tetrahydrofuran (2 ml) was added. Added thereto was 80 µl of 1,2-dibromoethane, the mixture was stirred under heating at 65°C for 1 minute, and further stirred at room temperature for 30 minutes. Then, 100 µl of trimethylchlorosilane was added, and the mixture was stirred at room temperature for 30 minutes. Thereafter, a solution of methyl-4-bromomethylbenzoate (2.290 g, 10 mmol) in distilled tetrahydrofuran (15 ml) was added at 0°C, and the mixture was stirred at 0°C for 3 hours and then cooled to -78°C. A solution of cuprous chloride (0.990 g, 10 mmol) and lithium chloride (0.848 g, 20 mmol) in distilled tetrahydrofuran (10 ml) was prepared in a 100-ml reactor and cooled to -78°C, and the above liquid mixture was added thereto at -78°C. After the temperature was elevated to -20°C, the mixture was stirred for 30 minutes, and then re-cooled to -78°C. Added to this mixture was a solution of (1S,5R,6R,7R)-2-diphenoxyphosphoryloxy-3-methylene-6-[(1E,4R)-4-methyl-4-hydroxy-1-octenyl]-7-t-butyldimethylsilyloxybicyclo[3.3.0]octane 1b (0.640 g, 1 mmol) in dry tetrahydrofuran (10 ml) at -78°C, and the mixture was stirred at -78°C for 2 hours. Added to the liquid mixture was 200 ml of a saturated ammonium chloride aqueous solution to terminate the reaction, and the reaction mixture was extracted with ethyl acetate (200 ml x 2 times). A separated organic layer was washed with a sodium chloride aqueous solution, dried over anhydrous magnesium sulfate, and concentrated to give a crude product (2.10 g). This product was subjected to silica gel chromatography (silica gel 40 g; elution liquid hexane:ethyl acetate = 20:1 to 10:1) to give an intended compound 4b (0.496 g, 0.88 mmol, yield 88%).

| | |
|---|---|
| ¹H-NMR (CDCl₃, ppm) δ: | 7.95 (2H,d,J=7.5Hz), 7.20(2H,d,J=7.5Hz), 5.45(2H,m), 5.25(1H,d,J=1.2Hz), 3.90(3H,s), 3.70(1H,m), 3.00(1H,m), 2.80(2H,t,7=7.5Hz), 2.40-1.90(8H,m), 1.60-1.10(9H,m), 1.15(3H,s), 1.95-1.80(9H,m), 0.09(9H,s) |
| IR (liquid film) cm⁻¹: | 3340, 2956, 2930, 2915, 1682, 1611, 965, 735 |

### Reference Examples 3-4

Reactions of reactants 1b (1 mmol) and 1c (1 mmol) each with a zinc compound (10 mmol) prepared from methyl-3-iodobenzoate (10 mmol) were carried out in the presence of CuCl (10 mmol) and LiCl (20 mmol) in the same manner as In Reference Example 1. The reaction mixtures were each subjected to post-treatment and subsequent column-separation to give corresponding products 4c and 4d at yields shown in Table 1. The NMR spectra of the products shown in Table 2 show that all the products were almost intended positional isomers and the reactions proceeded with highly positional selectivity.

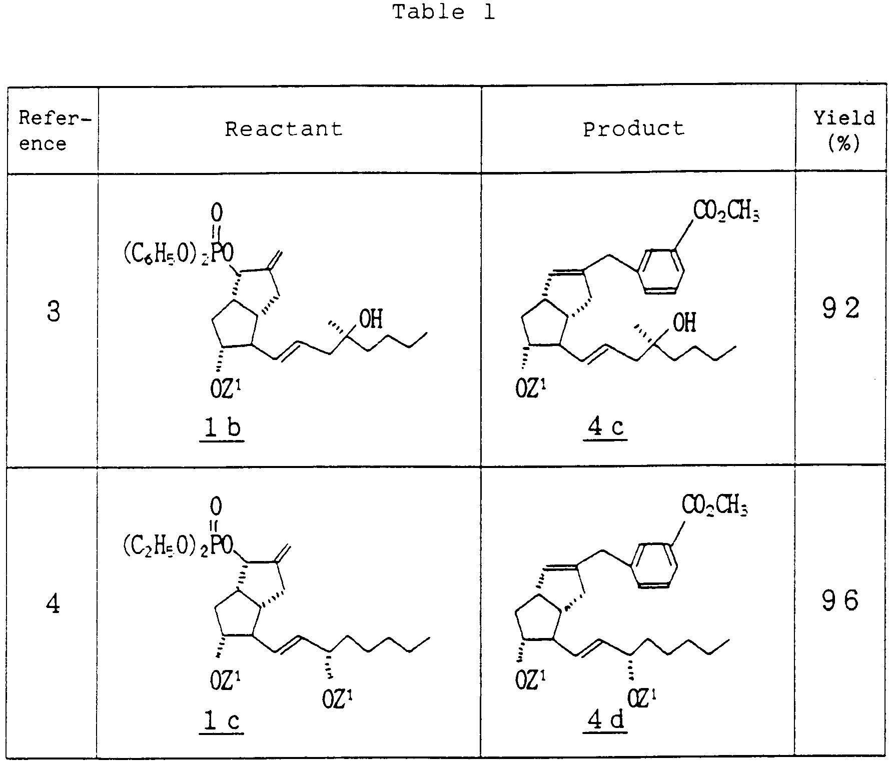

**Table 2**

| Reference | Product | ¹H-NMR (CDCl₃, ppm) δ |
|---|---|---|
| 3 | 4c | 7.85 (2H,m), 7.38 (2H,m), 5.45 (2H,m), 5.35 (1H,d,J=1.2Hz), 3.90 (3H,s), 3.78 (1H,m), 3.40 (2H,s), 3.00 (1H,m), 2.40∼1.85 (8H,m), 1.60 (1H,s), 1.50∼1.20 (6H,m), 1.15 (3H,s), 1.00∼0.80 (12H,m), 0.10∼0.00 (6H,m) |
| 4 | 4d | 7.85 (2H,m), 7.38 (2H,m), 5.40 (2H,m), 5.30 (1H,d,J=1.2Hz), 4.10 (1H,m), 3.85 (3H,s), 3.75 (1H,m), 3.30 (2H,s), 2.95 (1H,m), 2.40∼2.20 (3H,m), 1.95∼1.80 (2H,m), 1.65∼1.20 (9H,m), 0.95∼0.85 (21H,m), 0.10∼0.00 (12H,m) |
| 4c: IR (liquid film) cm⁻¹: | | |
| 3340, 2956, 2932, 1715, 1607, 965, 735 | | |
| 4d:_IR (liquid film) cm⁻¹: | | |
| 2956, 2932, 1715, 1607, 965, 735 | | |

### Reference Examples 5-6

Reactions of reactants 1a (1 mmol) and 1c (1 mmol) each with a zinc compound (10 mmol) prepared from methyl-4-(bromomethyl)benzoate (10 mmol) were carried out in the presence of CuCl (10 mmol) and LiCl (20 mmol) in the same manner as in Reference Example 2. The reaction mixtures were each subjected to post-treatment and subsequent column-separation to give corresponding products 4e and 4f at yields shown in Table 3. Table 4 shows the analytical results thereof.

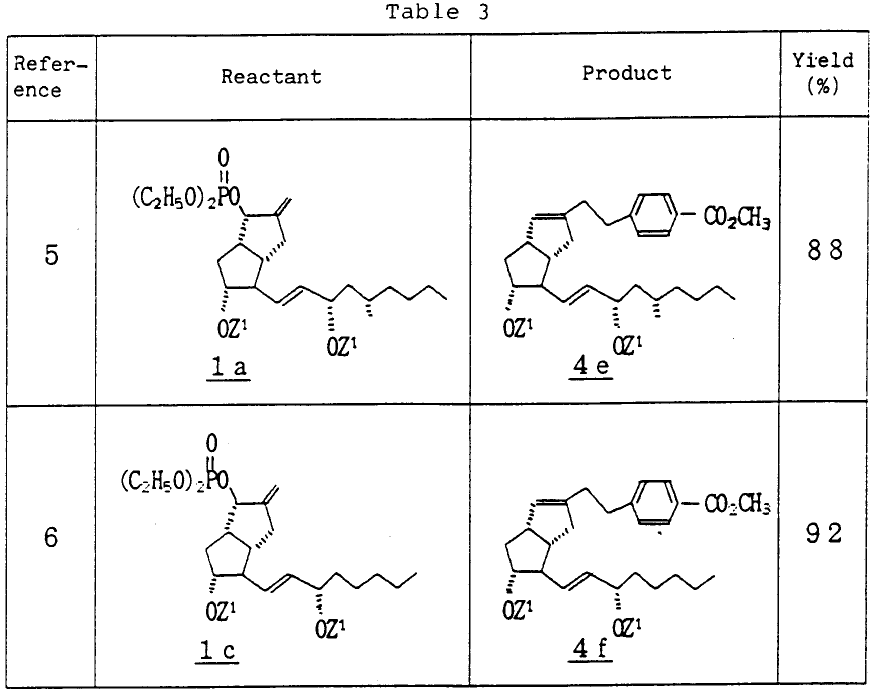

**Table 4**

| Reference | Product | ¹H-NMR (CDCl₃, ppm) δ |
|---|---|---|
| 5 | 4e | 7.95 (2H,d,J=7.5Hz), 7.25 (2H,d,J=7.5Hz), 5.50 (2H,m), 5.25 (1H,d,J=1.2Hz), 4.15 (1H,m), 3.85 (3H,s), 3.85 (1H,m), 3.00 (1H,m), 2.80 (2H,t,J=8Hz), 2.40∼1.80 (7H,m), 1.65∼1.20 (10H,m), 0.90∼0.85 (24H,m), 0.10∼0.00 (12H,m) |
| 6 | 4f | 7.95 (2H,d,J=7.5Hz), 7.25 (2H,d,J=7.5Hz), 5.50 (2H,m), 5.25 (1H,d,J=1.2Hz), 4.15 (1H,m), 3.85 (3H,s), 3.85 (1H,m), 3.00 (1H,m), 2.80 (2H,t,J=8Hz), 2.40∼1.80 (7H,m), 1.65∼1.20 (9H,m), 0.90∼0.85 (21H,m), 0.10∼0.00 (12H,m) |
| 4e: IR (liquid film) cm⁻¹ | | |
| 2955, 2920, 1724, 1607, 1590, 965, 735 | | |
| 4f: IR (liquid film) cm⁻¹: | | |
| 2955, 2920, 1724, 1607, 1590, 965, 735 | | |

### Reference Example 7

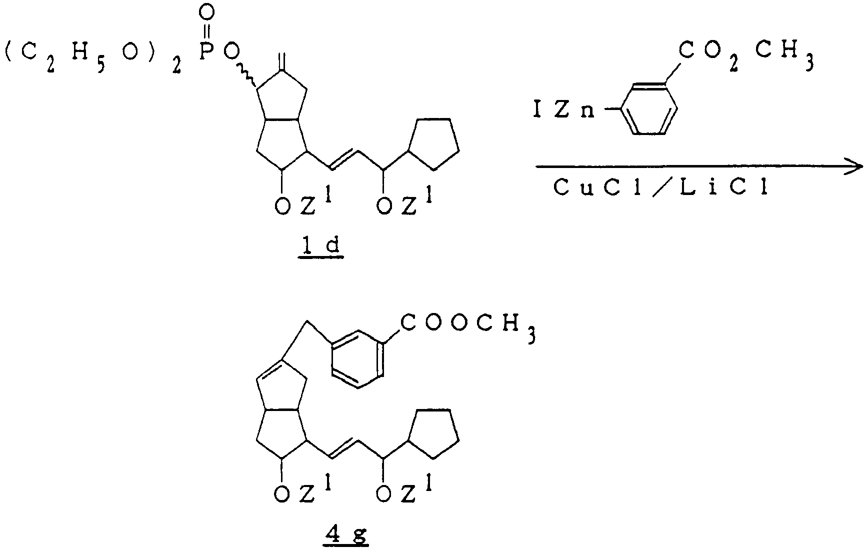

A reaction of a reactant 1d (1 mmol) with a zinc compound (10 mmol) prepared from methyl-3-iodobenzoate (10 mmol) was carried out in the presence of CuCl (10 mmol) and LiCl (20 mmol) in the same manner as in Reference Example 1. The reaction mixture was subjected to post-treatment and subsequent column-separation to give a corresponding product 4g (yield 86%).

### Reference Example 8

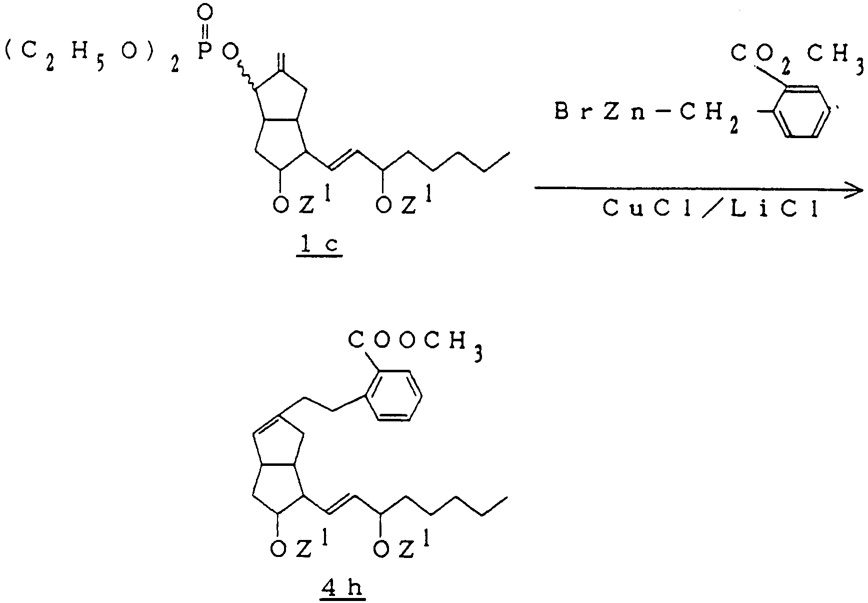

A reaction of a reactant 1c (1 mmol) with a zinc compound (10 mmol) prepared from methyl-2-(bromomethyl)benzoate (10 mmol) was carried out in the presence of CuCl (10 mmol) and LiCl (20 mmol) in the same manner as in Reference Example 2. The reaction mixture was subjected to post-treatment and subsequent column-separation to give a corresponding product 4h (yield 86%).

### Reference Example 9

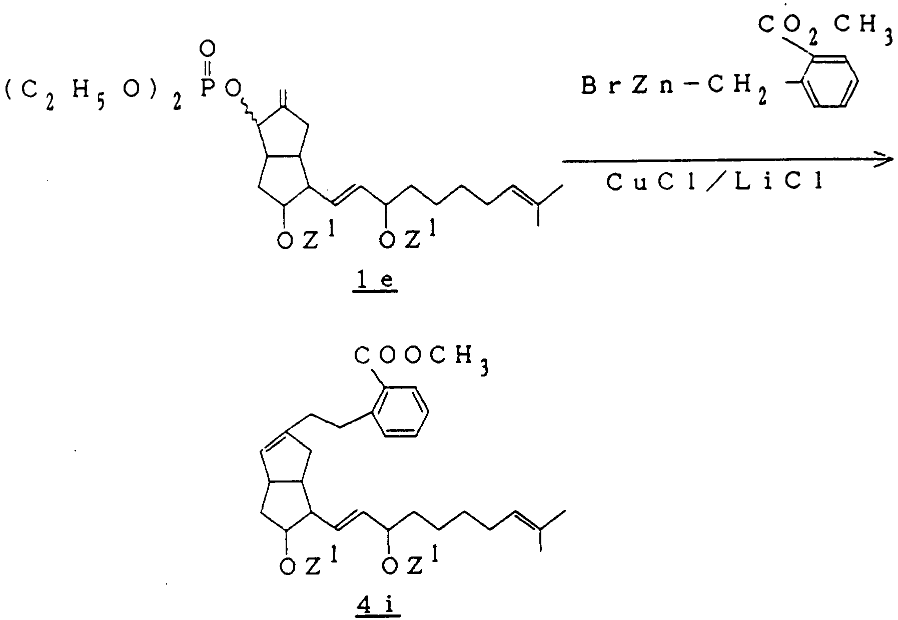

A reaction of a reactant 1e (1 mmol) with a zinc compound (10 mmol) prepared from methyl-2-(bromomethyl)benzoate (10 mmol) was carried out in the presence of CuCl (10 mmol) and LiCl (20 mmol) in the same manner as in Reference Example 2. The reaction mixture was subjected to post-treatment and subsequent column-separation to give a corresponding product 4i (yield 86%).

### Reference Example 10

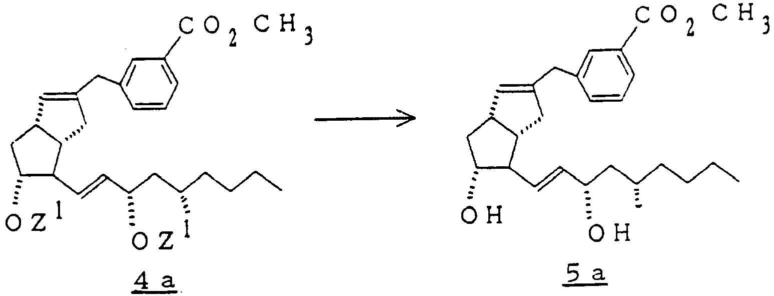

A disilyl compound 4a (950 mg, 1.45 mmol) was dissolved in 10 ml of THF, the mixture was stirred under cooling with ice, and 6 ml (2.0 equivalent) of a 1 M tetrabutylammonium fluoride solution in THF was added. The mixture was warmed to 30°C, and stirred for 5 hours. The reaction solvent, THF, was distilled off under reduced pressure, a saturated ammonium chloride aqueous solution and ethyl acetate were added to the concentrate, and the mixture was subjected to extraction. An organic layer was washed with a saturated sodium chloride aqueous solution, dehydrated, and dried over anhydrous magnesium sulfate. Then, the solvents were distilled off under reduced pressure to give a concentrate. The concentrate was subjected to silica gel chromatography (n-hexane:ethyl acetate = 1:1 to 1:2) for separation and purification to give 586 mg (yield 95%) of a diol 5a.

| | |
|---|---|
| ¹H-NMR (CDCl_{3'} ppm) δ: | 7.85(2H,m), 7.35(2H,m), 5.40(2H,m), 5.30(1H,d,J=1.2Hz), 4.10(1H,m), 3.90(3H,s) 3.70(1H,m), 3.38(2H,bs), 3.00(1H,m), 2.40-2.20(3H,m), 1.95-1.80(2H,m), 1.40-1.10(12H,m), 0.85(6H,m) |
| IR (liquid film) cm⁻¹: | 3364, 2955, 2920, 2872, 1725, 1607, 1590, 1446, 1435, 1281, 1200, 1105, 1088, 995, 970, 756, 704 |
| Mass m/e: | 408(M⁺-H₂O) |
| UV λ C₂H₅OH max nm (log ε): | 231.6 (3.94) |

### Reference Examples 11-18

In the same manner as in Reference Example 10, reactants 4b, 4c, 4d, 4e, 4f, 4g, 4h and 4i in an amount of 1 mmol each were respectively dissolved in 10 ml of THF, each mixture was stirred under cooling with ice, and 4 ml (2.0 equivalent) of a 1 M tetrabutylammonium fluoride solution in THF was added. Each mixture was warmed to 30°C, and stirred for 5 hours. The reaction mixtures were independently treated in the same manner as in Reference Example 10, and the obtained crude products were subjected to silica gel chromatography (n-hexane:ethyl acetate = 1:1 to 2:1) for separation and purification to give corresponding diol compounds 5b, 5c, 5d, 5e, 5f 5g, 5h and 5i at yields shown in Table 5.

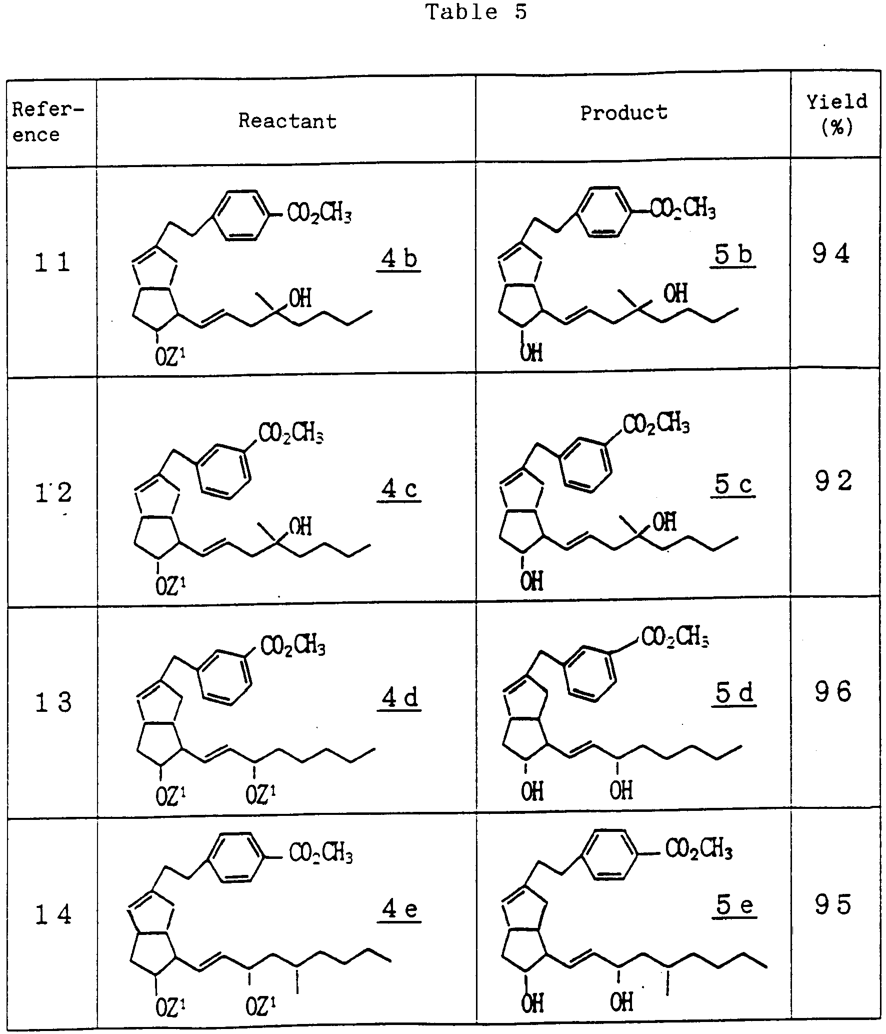

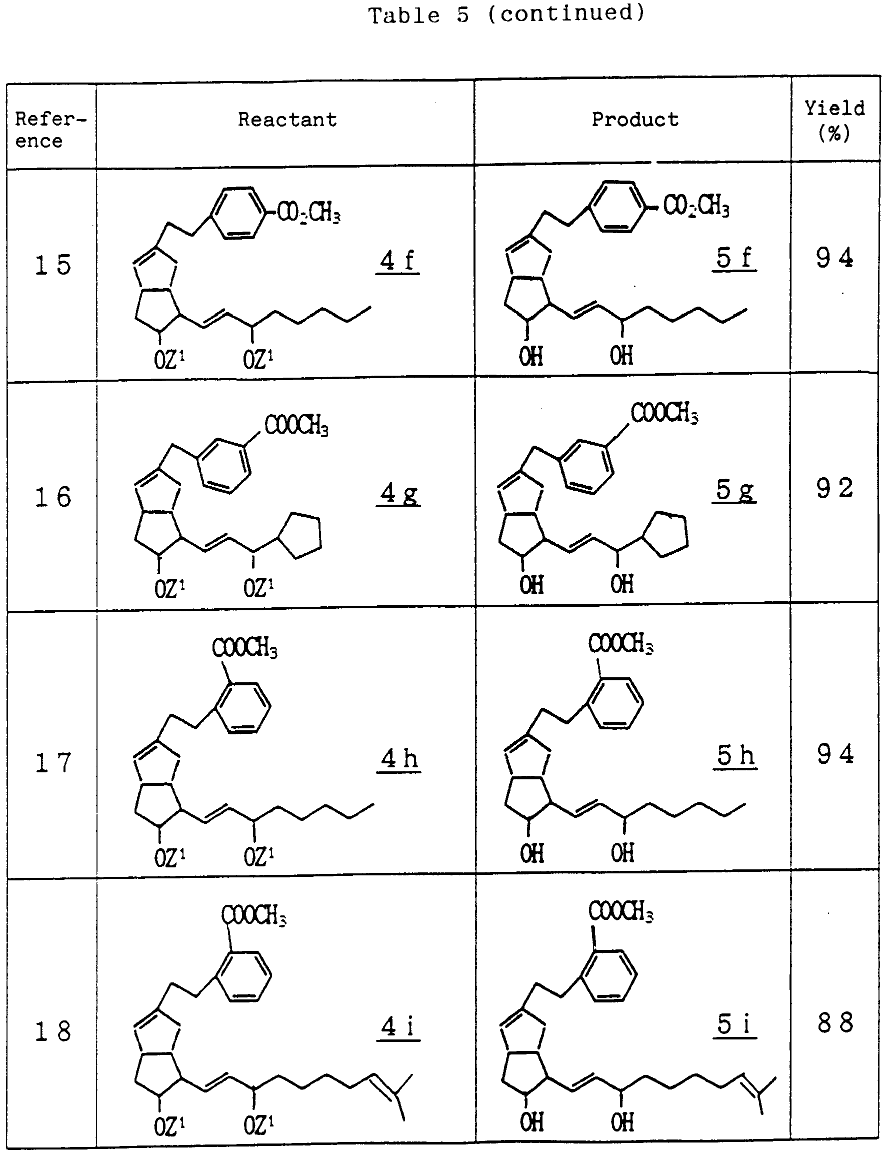

Table 6 shows the physical values of the compounds 5b, 5c, 5d, 5e, 5f, 5g, 5h and 5i.

**Table 6**

| Compound | ¹H-NMR (CDCl₃. ppm) δ | IR (liquid film) cm⁻¹ | UV λ.C₂H₅0H max nm (log *ε* ) | | MS |
|---|---|---|---|---|---|
| 5b | 7.95(2H,d,J=7.5Hz), 7.25(2H,d,J=7.5Hz), 5.50(2H,m), 5.30(1H.d.J=1.2Hz), 3.90(3H,s), 3.75(1H,m), 3.00(1H.m). 2.80(2H,t,J=8Hz). 2.40-1.20(18H.m), 1.15(3H,s), 0.90(3H,t) | 3380, 2953, 2932, 2872, 1723, 1611, 1455, 1435, 1310, 1281, 1192, 1179, 1113. 1021, 909, 768, 733, 708 | 239.2 nm, (4.16) | 208.6 nm (4.04) | M⁺=426(No detct.) M/e=408M⁺H₂O |
| 5c | 7.85(2H.m), 7.40(2H.m). 5.45(2H,m), 5.30(1H,d,J=1.2Hz), 3.90(3H.s). 3.75(1H.m), 3.40(2H.bs). 3.00(1H.m). 2.40-1.15(16H.m), 1.10(3H,s). 0.90(3H.t) | 3384, 2955. 2932. 2872, 1725, 1607, 1590, 1447, 1439. 1379, 1283, 1200, 1107, 1090, 992. 974, 758 | - | - | M⁺=412(No detct.) M/e=394=M⁺H₂O |
| 5d | 7.85(2H,m), 7.38(2H.m). 5.50(2H,m), 5.30(1H.d.J=1.2Hz), 4.05(1H,m), 3.90(3H,s), 3.75(1H,m), 3.38(2H,bs), 3.00(1H.m). 2.40-1.15(16H.m), 0.85(3H,t) | 3370. 2923, 1720, 1701, 1445, 1438, 1307, 1281. 1202, 1113, 1086, 972, 760 | 231.6 nm, (3.94) | 211.2 nm (3.97) | |
| 5e | 7.95(2H,d,J=7.50Hz), 7.25(2H.d.J=7.5Hz). 5.55(2H.m). 5.32(1H.d.J=1.2Hz). 4.18(1H,m), 3.90(3H.s), 3.75(1H.m). 3.00(1H,m), 2.80(2H,t,J=8Hz), 2.40-1.20(19H.m). 0.90(6H.m) | 3400, 3335, 2955, 2928, 1728, 1723, 1717, 1705, 1611, 1456, 1485. 1416, 1377. 1310, 1192. 1179. 1111, 1021. 968, 911, 856. 768, 733 | 238.8 nm, (4.07) | 209.0 nm (3.95) | M⁺=440(No detct.) M/e=422=M⁺H₂O |
| 5f | 7.95(2H.d.J=10Hz). 7.36(2H.d,J=7.5Hz), 5.55(2H,m), 5.30(1H.d,J=1.2Hz), 4.05(1H,m), 3.90(3H.s). 3.75(1H,m), 3.00(1H.m). 2.80(2H.t.J=8Hz), 2.40-1.15(18H.m). 0.90(3H,t) | 3524. 3425. 2953, 2928, 2863, 1720. 1701, 1610, 1437, 1416. 1285. 1194. 1179. 1113. 1086, 1020, 972, 766 | 239.2 nm, (3.92) | 210.2 nm (3.80) | |

### Reference Example 19

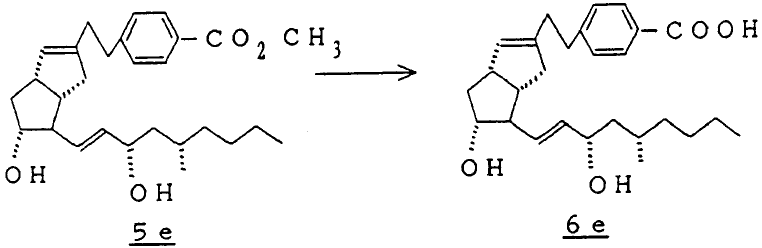

A diol 5e in an amount of 67.5 mg (0.15 mmol) was dissolved in 6 ml of THF at room temperature, an alkali aqueous solution of 40 mg (0.95 mmol) of LiOH · H₂O and 3 ml of H₂O was dropwise added to the THF solution with stirring, and the mixture was further stirred for 26 hours to show that the starting materials disappeared on TLC [hexane:ethyl acetate = 1:4]. Added to the liquid mixture was 2 ml of 1 N-HCl, and the mixture was stirred for 30 minutes. Then, a saturated sodium chloride aqueous solution and ethyl acetate were added to the reaction mixture, and the mixture was subjected to extraction. An organic layer was rewashed with a saturated sodium chloride aqueous solution, dehydrated and dried over anhydrous magnesium sulfate, and the solvent was distilled off under reduced pressure. The obtained concentrate was subjected to silica gel chromatography [hexane:ethyl acetate = 1:2 to 1:4 (containing 0.1% acetic acid)] for separation and purification to give 57 mg (yield 89%) of a carboxylic acid 6e.

| | |
|---|---|
| ¹H-NMR (CDCl₃, ppm) δ: | 8.00(2H,d,J=7.5Hz), 7.25(2H,d,J=7.5Hz), 6.00(3H,bs), 5.45(2H,m), 5.25(1H,d,J=1.2Hz), 4.15(1H,m), 3.75(1H,m), 3.00(1H,m), 2.75(2H,t,-J=7.5Hz), 2.40-1.80(7H,m), 1.45-1.20(10H,m), 0.85(6H,m) |
| IR (liquid film) cm⁻¹: | 2955, 2924, 2872, 1692, 1613, 1424, 1318, 1289, 1179, 1090, 1015, 968, 839, 762, 708, 673 |
| Mass m/e: | 408(M⁺-H₂O) |
| UV λ C₂H₅OH max nm (log ε): | 237.8 (4.38), 207.8 (4.29) |

### Reference Example 20

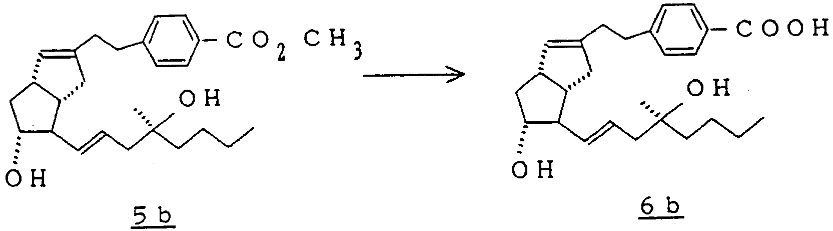

The hydrolysis of 100 mg (0.24 mmol) of a reactant 5b was carried out in the same manner as in Reference Example 19. The reaction product was subjected to post-treatment and subsequent column-purification to give 91 mg (yield 96%) of a corresponding carboxylic acid 6b.

| | |
|---|---|
| ¹H-NMR (CD₃OD, ppm) δ : | 8.00(2H,d,J=7.5Hz), 7.25(2H,d,J=7.25Hz), 6.00(3H,bs), 5.55(1H,m), 5.42(1 H,m), 5.25(1H,d,J=1.2Hz), 3.75(1H,m),3.00(1H,m), 2.80(2H,t,-J=10Hz), 2.60-1.80(9H,m), 1.50-1.20(7H,m), 1.15(3H,s), 0.90(3H,t) |
| IR (liquid film) cm⁻¹: | 2956, 2930, 2915, 2842, 2664, 1682, 1611 1576, 1455, 1424, 1320, 1291, 1179, 1090, 972, 847, 762, 706, 677 |
| Mass m/e: | 394(M⁺-H₂O) |
| UV λ C₂H₅OH max nm (log ε): | 237.8 (4.16), 208.0 (4.08) |

### Reference Example 21

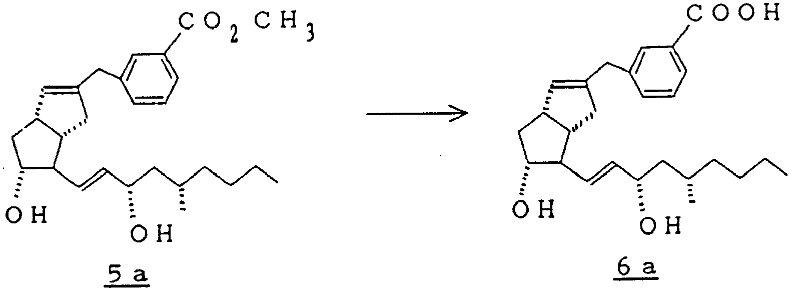

The hydrolysis of 190 mg (0.446 mmol) of a reactant 5a was carried out in the same manner as in Reference Example 19. The reaction product was subjected to post-treatment and subsequent column-purification to give 184 mg (yield 100%) of a corresponding carboxylic acid 6a.

| | |
|---|---|
| ¹H-NMR (CD₃OD, ppm) δ : | 7.85(2H,m), 7.40(2H,m), 5.65-5.30(3H,m), 4.05(1H,m), 3.75(1H,m), 3.45(2H,bs), 3.30(1 H,m), 3.00(1 H,m), 2.40-2.15(17H,m), 0.85(6H,m) |
| Mass m/e: | 394(M⁺-H₂O) |
| IR (liquid film) cm⁻¹: | 3360, 2957, 2928, 2645, 1696, 1607, 1589, 1453, 1412, 1377, 1279, 1196, 1086, 997, 970, 839, 816 |
| UV λ C₂H₅OH max nm (log ε): | 229.6 (3.94), 213.0 (3.95) |
| UV λ CH₃OH max nm (log ε): | 229.6 (3.94), 213.0 (3.95) |

### Reference Examples 22-24

Hydrolyses of 1 mmol of each of reactants 5d, 5f and 5g were carried out in the same manner as in Reference Example 19. The reaction products were independently subjected to post-treatment and subsequent column-purification to give corresponding carboxylic acids 6d, 6f and 6g at yields shown in Table 7.

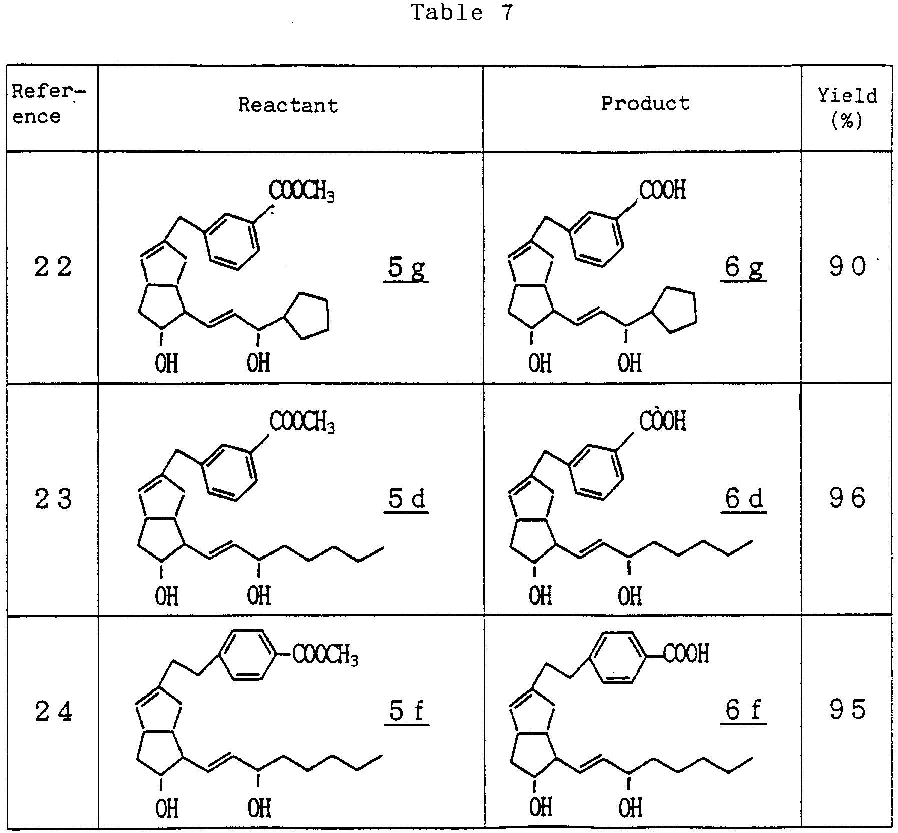

Table 8 shows the physical property values of the compounds 6d, 6f and 6g.

**Table 8**

| Compound | ¹H-NMR (CD₃OD, ppm) δ | IR (liquid film) cm⁻¹ | UV λC₂H_{S}OH max nm (log ε) |
|---|---|---|---|
| 6g | 7.85(2H,m), 7.35(2H,m), | 3300, 2955, 2920, 1720, 1607, 1590, | -- |
| | 5.60-5.30(3H,m), 4.10(1 H,m), 3.70(1 H,m), | 1255, 1100, 1000, 970, 835, 770 | |
| | 3.38(2H,bs), 3.00(1 H,m), | | |
| | 2.50-1.10(18H,m) | | |
| 6d | 7.85(2H,m), 7.35(2H,m), 5.55-5.25(3H,m), | 3300, 2957, 1733, 1717, 1700, 1609, | 229.6 nm, 215.0 nm |
| | 4.95(2H,m), 4.20(1 H,m), 3.90(1 H,m), | 1590, 1456, 1194, 968, 752 | (3.97) (3.95) |
| | 3.65(1 H,m), 3.40(2H,bs), 3.00(1 H,m), | | |
| | 2.40-1.15(15H,m), 0.85(3H,t) | | |
| 6f | 7.95(2H,d,J=7.5Hz), 7.30(2H,d,J=7.5Hz), | 3325, 2928, 1734, 1717, 1698, 1684, | 238.0 nm, 208.6 nm |
| | 5.55(2H,m), 5.30(1 H,d,J=1.2Hz), | 1636, 1589, 1490, 1456, 1420, 1339, | (4.09) (3.94) |
| | 4.00(1 H,m), 3.70(1 H,m), 2.95(1 H,m), | 1293, 1179, 1084, 972, 853 | |
| | 2.80(2H,t,J=8Hz), 2.45-1.15(18H,m), | | |
| | 0.85(3H,t) | | |

### Example 1

### Measurement of the inhibition activity on cell migration:

To investigate the inhibition activity on cell migration of a compound to be tested, cell migration mediated by human platelet derived growth factor (PDGF) is measured by using vascular smooth muscle cell derived from a human normal aorta as the migration cell and according to the method of McCarthy et al. (J. Cell. Biol., 97, 772-777(1983)) as shown bellow.

A Transwell Chamber (Costar Co., registered trademark) was used to measure the inhibitory activity. The chamber is divided into a lower and a upper chamber with an 8-µm pore size membrane filter (PVP-free polycarbonate filter, Nucleopore Co., registered trademark), and the filter was pretreated on its lower surface with 5 µg of rat tail collagen type 1 (Becton Dickinson Co.). To the upper chamber was added 100 µl of a 1.5 x 10⁶/ml suspension of cells in a mixed solution of DMEM (supplied by Flow Laboratories Co.) and 0.1% BSA (supplied by Nakarai Tesque Co.), and a solution of human PDGF (supplied by Becton Dickinson Co.), which is prepared by diluting with the same mixed solution to giving the final concentration of 10 ng/ml, is filled in the lower chamber (600 µl). At the same time, each of the test compounds was added both into the upper chamber and the lower chamber at various testing concentrations. After the chamber was allowed to stand under 5%-CO₂ atmosphere at 37°C For 6 hours, the filter was taken out. It was fixed in 99.7% methanol, and stained with hematoxylin and eosin. After cells adhered on the upper surface of the filter were removed with a cotton swab, the cells migrated to the lower surface were counted under a high-magnifying-power microscope (x200, x400) in terms of cell nuclei. Usually, five fields were counted per filter. Cell migration was expressed by the average cell count of the five fields ± the standard deviation. The significant difference was examined by Student's two-tailed t-test.

From the test result, it was found that the compound 5e and 6b have the inhibition activities on cell migration of 13.9 % and 20.0% at the concentration of 10⁻⁷M compared with the cell migration counts in the case where no test compound was added respectively. Further, it was found that neither of the compounds 5e and 6b had problem with acute toxicity

### Example 2

Tablets each consisting of the following composition were prepared.

| | |
|---|---|
| Active ingredient | 200 µg |
| Lactose | 180 mg |
| Potato starch | 50 mg |
| Polyvinylpyrrolidone | 10 mg |
| Magnesium stearate | 5 mg |

The active ingredient, lactose and potato starch were mixed, and the mixture was homogeneously moistened with 20% polyvinylpyrrolidone solution in ethanol. Subsequently, this was passed through a 20-mm mesh sieve, dried at 45°C, and then passed once again through a 15-mm mesh sieve. The obtained grains were mixed with magnesium stearate, and compressed into tablets.

As the active ingredient, (1S,5S,6S,7R)-3-(p-carboxybenzyl)-6-[(1E,4S)-4-hydroxy-4-methyl-1-octenyl]-7-hydroxybicyclo[3.3.0]-2-octene methyl ester was used.

### Example 3

Hard gelatin capsules each containing the following composition were prepared.

| | |
|---|---|
| Active ingredient | 100 µg |
| Microcrystalline cellulose | 195 mg |
| Amorphous silicic acid | 5 mg |

The active component, microcrystalline cellulose and unpressed amorphous silicic acid were sufficiently mixed, and the mixture was filled into hard gelatin capsules. In a typical case, the same compound as in Example 2 was used as the active ingredient.

### Industrial Field of Application

A preparation for inhibiting vascular smooth muscle cell migration of the present invention containing an isocarbacyclin expressed by formula (1) and/or its enantiomer as an active ingredient is useful for inhibiting the hypertrophy and occlusion of a blood vessel caused mainly by vascular smooth muscle cell migration, for example, after various angioplastic operations, arterial bypass operations and organic transplantations and the like. The preparation is useful also as an agent for prevention and therapy of the hypertrophy and occlusion of a blood vessel, and further useful as an agent for prevention and therapy of arteriosclerosis.

## Claims

1. A vascular smooth-muscle-cell migration inhibitor containing an isocarbacyclin of the following formula (1), 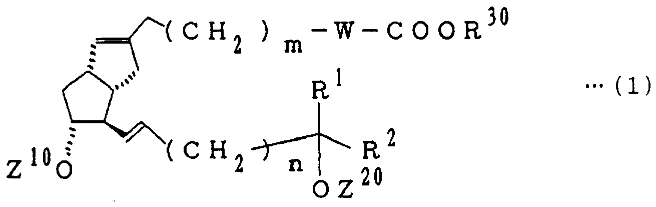 wherein W is an ortho, meta or para phenylene group, a C₃-C₇ cycloalkylene group or a thiophendiyl group; R¹ is a hydrogen atom, a methyl group, an ethyl group or a vinyl group; R² is a linear or branched C₃-C₉ alkyl group, alkenyl group or alkynyl group, or a C₃-C₇ cycloalkyl group each of which is optionally substituted; R³⁰ is a hydrogen atom, a C₁-C₁₀ alkyl group, a phenyl group, a benzyl group, a naphthyl group or one equivalent of a cation; each of Z¹⁰ and Z²⁰ is independently a hydrogen atom, a tri(C₁-C₇ hydrocarbon)silyl group or a group which forms an acetal bond or an ester bond together with an oxygen atom to which it bonds; n is 0 or 1; and m is an integer of 0 to 4, and/or its enantiomer as an active ingredient.

2. A vascular smooth-muscle-cell migration inhibitor of claim 1, wherein, in the above formula (1), W is an ortho, meta or para phenylene group, and each of Z¹⁰ and Z²⁰ is a hydrogen atom at the same time.

3. A vascular smooth-muscle-cell migration inhibitor of claim 1, wherein, in the above formula (1), R¹ is a hydrogen atom or a methyl group, R² is a linear or branched C₃-C₉ alkyl group or a C₃-C₉ alkyl group substituted with a C₃-C₆ cycloalkyl group, R³⁰ is a hydrogen atom, a linear or branched C₁-C₁₀ alkyl group or one equivalent of a cation, and m is 0 or 1.

4. A vascular smooth-muscle-cell migration inhibitor of claim 2, wherein, in the above formula (1), R¹ is a hydrogen atom or a methyl group, R² is a linear or branched C₃-C₉ alkyl group or a C₃-C₉ alkyl group substituted with a C₃-C₆ cycloalkyl group, R³⁰ is a hydrogen atom, a linear or branched C₁-C₁₀ alkyl group or one equivalent of a cation, and m is 0 or 1.

5. An agent for the prevention or therapy of post-PTCA restenosis containing an isocarbacyclin of the above formula (1) and/or its enantiomer as active ingredient.

6. An agent for the prevention or therapy of post-PTCA restenosis of claim 5, wherein, in the above formula (1), W is an ortho, meta or para phenylene group, and each of Z¹⁰ and Z²⁰ is a hydrogen atom at the same time.

7. An agent for the prevention or therapy of post-PTCA restenosis of claim 5, wherein, in the above formula (1), R¹ is a hydrogen atom or a methyl group, R² is a linear or branched C₃-C₉ alkyl group or a C₃-C₉ alkyl group substituted with a C₃-C₆ cycloalkyl group, R³⁰ is a hydrogen atom, a linear or branched C₁-C₁₀ alkyl group or one equivalent of a cation, and m is 0 or 1.

8. An agent for the prevention or therapy of post-PTCA restenosis of claim 6, wherein, in the above formula (1), R¹ is a hydrogen atom or a methyl group, R² is a linear or branched C₃-C₉ alkyl group or a C₃-C₉ alkyl group substituted with a C₃-C₆ cycloalkyl group, R³⁰ is a hydrogen atom, a linear or branched C₁-C₁₀ alkyl group or one equivalent of a cation, and m is 0 or 1.
